# FASCICULE DE BREVET EUROPEEN

(11) **EP 1 589 010 B1**
(45) Date de publication et mention de la délivrance du brevet: **11.06.2008**
(21) Numéro de dépôt: 05290793.8
(22) Date de dépôt: 11.04.2005
(51) Int. Cl.: C07D 309/10, A61K 31/70, C07H 17/08, A61K 8/60

(54) **Nouveaux dérivés C-glycosides et utilisations cosmétiques**
Neue C-glukoside-Derivate und ihre kosmetische Verwendungen
New C-Glycoside derivatives and uses thereof

(30) Priorité: 23.04.2004 FR 0450773
(43) Date de publication de la demande: 26.10.2005
(73) Titulaire: L'ORÉAL, 75008 Paris (FR)
(72) Inventeur: Trouille, Simon, 60290 Rantigny (FR); Cavezza, Alexandre, 93320 Pavillon sous Bous (FR); Pichaud, Patrick, 91190 Gif-sur-Yvette (FR)
(74) Mandataire: Kromer, Christophe

(56) Documents cités:
- WO-A-02/051803
- WO-A-02/051828

## Description

La présente invention concerne un procédé de synthèse, de nouveaux dérivés C-glycosides de configuration absolue donnée.

Certains dérivés C-glycosides ont démontré des propriétés biologiques intéressantes, en particulier pour lutter contre le vieillissement de l'épiderme, et/ou contre le dessèchement de la peau. C'est notamment le cas des composés décrits dans la demande de brevet WO-02/051828 déposée au nom de la Demanderesse. Ces composés agissent par l'intermédiaire de la stimulation de la synthèse des glycosaminoglycannes contenant un résidu D-glucosamine et/ou N-acétyl-D-glucosamine, et sont représentés par la formule (F) : 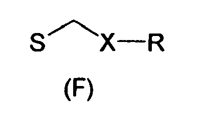 dans laquelle S représente un monosaccharide ou un polysaccharide, R représente différents radicaux linéaires ou cycliques, et le groupement X peut représenter un groupement choisi parmi : -CO-, -CH(NR₁R₂)-, -CHR'-, -C(=CHR')-, R' pouvant représenter différents radicaux, dont le radical hydroxyle.

Dans le cas où R' est un radical hydroxyle, le composé de formule (F) correspondant répond à la formule (F2) : 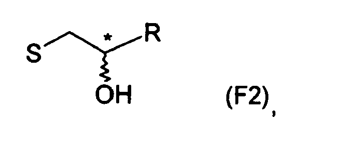 et présente un carbone asymétrique, pouvant exister sous deux formes épimères R, et S.

Considérant également que le sucre S peut être relié à la chaîne latérale par une liaison α ou β, le composé qui en résulte de formule (F3) : 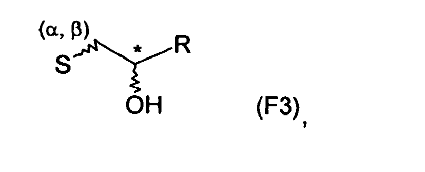 peut se présenter sous la forme de 4 diastéréoisomères désignés : (α, S) ; (α, R) ; (β, S) ; et (β, R).

Les composés de formule (F3) ont été décrits jusqu'alors sous forme de mélanges de plusieurs diastéréoisomères. En particulier la demande WO-02/051828 divulgue un procédé de synthèse permettant de conduire à ces dérivés sous la forme d'un mélange de diastéréoisomères :

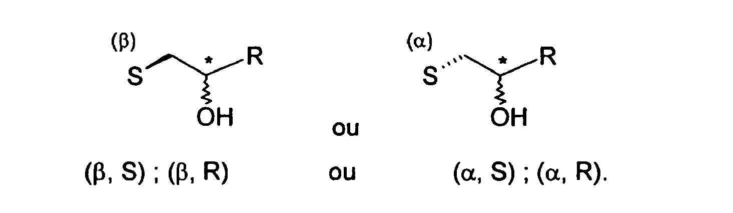

Aucune méthode n'a permis de conduire à l'obtention de l'une des formes diatéréoisomériquement pure.

La Demanderesse a mis en évidence que l'un de ces diastéréoisomères, (β, S), pouvait être obtenu de façon sélective, et des études ont montré qu'il possédait une activité biologique bien supérieure à celle des composés décrits sous forme de mélange dans la demande WO-02/051828.

La présente invention concerne donc un procédé de synthèse de composés C-glycosides de formule (I): 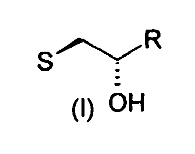 dans laquelle,
- S représente un monosaccharide ou un polysaccharide comprenant jusqu'à 20 unités sucre, de préférence jusqu'à 6 unités sucre, sous forme pyranose et/ou furanose et de série D, ledit mono- ou polysaccharide présentant au moins une fonction hydroxyle obligatoirement libre et éventuellement une ou plusieurs fonctions amine éventuellement protégée,
- la liaison 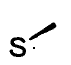 représente une liaison de configuration β,
- R représente une chaîne alkyle, perfluoroalkyle, hydrofluoroalkyle linéaire ou ramifiée, saturée ou insaturée comprenant de 1 à 18 atomes de carbone, un cycle cycloalkyle, cycloperfluoroalkyle, cyclohydrofluoroalkyle comprenant de 3 à 11 atomes de carbone saturé ou insaturé, un radical phényle ou benzyle, ladite chaîne ou ledit cycle pouvant être éventuellement interrompu par un ou plusieurs hétéroatomes choisi parmi l'oxygène, le soufre, l'azote, le silicium, et éventuellement substitué par au moins un radical choisi parmi -OR'₁, -SR"₁, -NR"'₁R'₂, -COOR"₂, -CONHR"'₂, -CN, halogène, perfluoroalkyle, hydrofluoroalkyle comprenant de 1 à 18 atomes de carbone, un radical cycloalkyle comprenant de 3 à 11 atomes de carbone, aryle, hétérocyclique éventuellement substitués,
- R'₁, R'₂, R"₁, R"₂, R"'₁, R"'₂, identiques ou différents, représentent un atome d'hydrogène, un radical choisi parmi un radical alkyle, hydroxyle, perfluoroalkyle et/ou hydrofluoroalkyle, linéaire ou ramifié, saturé ou insaturé, comprenant de 1 à 30 atomes de carbone,
ainsi que leurs sels physiologiquement acceptables.

Des composés de formule (I) sont connus de la publication « Preferred conformation of C-Glycosides. 1. Conformational similarity of glycosides and corresponding C-glycosides » Tse-Chong Wu, Peter G. Goekjian, Yoshito Kishi, J. Org. Chem, 1987, 52, p 4819-4823 : le C-β-D-glycopyranoside-2-(S),3-dihidroxypropane (appelé également le 2,6-anhydro-7-déoxy-L-gulo-nonitol) (RN 54548-38-8) et le C-β-D-(2-déoxyglycopyranoside)-2-(S),3-dihydroxypropane (appelé également le 4,8-anhydro-3,5-didéoxy-D-glycéro-D-gulo-nonitol) (RN 110352-39-1). Les composes nouveaux ( dits de formule (I)) correspondants aux composés de formule (I) décrites précédemment à l'exception des deux composés cités précédemment font donc partie de la présente invention.

Un aspect avantageux de l'invention concerne la synthèse de composés de formule (I) pour lesquels S représente un monosaccharide dont la fonction hydroxyle en position 3 est libre.

Avantageusement, les monosaccharides préférés sont choisi parmi le D-glucose, le D-galactose, le D-mannose, le D-xylose, le D-lyxose, l'acide D-glucuronique, l'acide D-galacturonique, l'acide D-iduronique, la N-acétyl-D-glucosamine, la N-acétyl- D-galactosamine. On préfèrera tout particulièrement le D-glucose, le D-xylose, ou la N-acétyl-D-glucosamine, et très préférentiellement le D-xylose.

Un autre aspect particulier de l'invention concerne la synthèse de composés de formule (I) pour lesquels S représente un polysaccharide tel que défini précédemment.

Avantageusement, les polysaccharides préférés contiennent jusqu'à 6 unités sucre et sont choisis parmi le D-maltose, le D-lactose, le D-cellobiose, le D-maltotriose, un disaccharide associant un acide uronique choisi parmi l'acide D-iduronique ou l'acide D-glucuronique avec une hexosamine choisi parmi la D-galactosamine, la D-glucosamine, la N-acétyl-D-galactosamine, la N-acétyl-D-glucosamine, un oligosaccharide contenant au moins un xylose avantageusement choisi parmi le xylobiose, le méthyl-β-xylobioside, le xylotriose, le xylotétraose, le xylopentaose et le xylohexaose et préférentiellement le xylobiose qui est composé de deux molécules de xylose liées par une liaison 1-4.

Un autre aspect particulier de l'invention concerne la synthèse de composés de formule (I) pour lesquels R représente une chaîne alkyle, perfluoroalkyle, hydrofluoroalkyle linéaire ou ramifiée, saturée ou insaturée comprenant de 1 à 6 atomes de carbone, un cycle cycloalkyle, cycloperfluoroalkyle, cyclohydrofluoroalkyle, comprenant de 3 à 11 atomes de carbone, ou un radical phényle ou benzyle, ladite chaîne ou ledit cycle pouvant être éventuellement interrompu par un ou plusieurs hétéroatomes choisi parmi l'oxygène, le soufre, l'azote, le silicium, et éventuellement substitué par au moins un radical choisi parmi -OR'₁, -SR"₁, -NR"'₁R'₂, -COOR"₂, -CONHR"'₂, -CN, halogène, perfluoroalkyle, hydrofluoroalkyle comprenant de 1 à 6 atomes de carbone, cycloalkyle comprenant de 3 à 11 atomes de carbone, aryle, hétérocyclique éventuellement substitués.

Avantageusement, R représente une chaîne alkyle linéaire ou ramifiée comprenant de 1 à 6 atomes de carbone ou un cycle cycloalkyle comprenant de 3 à 11 atomes de carbone. Préférentiellement, R représente une chaîne alkyle linéaire ou ramifiée comprenant de 1 à 6 atomes de carbone, de préférence de 1 à 4 atomes de carbones, et mieux de 1 à 3 atomes de carbone, et encore mieux de 1 à 2 atomes de carbone.

Parmi les groupements alkyle préférés, on peut citer les groupements méthyle, éthyle, propyle, isopropyle, butyle, isobutyle, ou tertbutyle, et préférentiellement un groupement méthyle.

Les groupements aryle préférés pour la mise en oeuvre de l'invention sont les groupements phényle et naphtyle.

Parmi les groupements hétérocycliques préférés pour la mise en oeuvre de la présente invention, on peut citer les groupements furyle, thiényle, pyrazolyle, imidazolyle, pyridyle, et pyrimidyle.

Parmi les composés C-glycosides de formule (1) on préfère tout particulièrement le C-β-D-xylopyranoside-2-(S)-hydroxy-propane de formule : 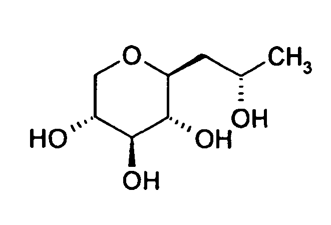 et le C-β-D-fucopyranoside-2-(S)-hydroxy-propane de formule :

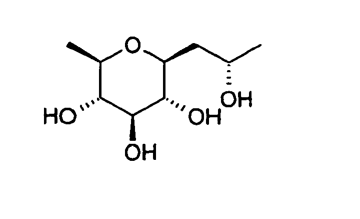

La présente invention concerne également le procédé de préparation des composés de formule (I) tels que définis précédemment. Ce procédé de préparation permet d'obtenir les composés de formule (I) de façon sélective sous la forme d'un seul diastéréoisomère (β, S).

Le composé de départ pour la synthèse du composé de formule (I) est le composé carbonylé correspondant de formule (II) : 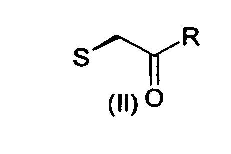 dans laquelle S et R ont la même signification que précédemment, et 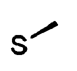 représente une liaison de configuration β.

Il est théoriquement possible de réaliser la réduction du composé (II) par des méthodes connues. Les réducteurs classiques peuvent être utilisés, comme par exemple :
- les hydrures d'aluminium, et leurs sels (en particulier leurs sels de lithium, de potassium, ou de sodium), ainsi que leurs dérivés comme par exemple le Red-AI (hydrure de sodium et de bis(2-méthoxyéthoxy)aluminium), ou le DIBAL-H (hydrure de diisobutylaluminium),
- les borohydrures, et leurs sels (en particulier leurs sels de lithium, de potassium, ou de sodium), ainsi que leurs dérivés comme par exemple les L-sélectride ou K-sélectride (triisobutylborohydrure de lithium ou de potassium respectivement),
- les métaux (par exemple le sodium, le potassium ou le lithium),
- l'hydrogénation catalytique en présence de catalyseurs classiques tels que le platine ou le palladium sur charbon.
Les solvants habituellement utilisés pour effectuer ces réactions de réduction sont choisis parmi les alcools primaires, secondaires ou tertiaires, linéaires ou ramifiés, et comportant entre 1 et 8 atomes de carbone (par exemple le méthanol, l'éthanol, l'isopropanol, le tertbutanol), les éthers linéaires ou cycliques (par exemple l'éther éthylique, le dioxane, ou le tétrahydrofurane), les composés aromatiques tels que le toluène, l'ammoniac liquide, et l'eau.

Ces procédés de synthèse décrits dans l'art antérieur conduisent toutefois à un mélange de deux formes diastéréoisomères dans des proportions variables, en partant d'un composé de formule (11) unique de configuration absolue fixée. C'est le cas notamment du procédé décrit dans la demande WO-02/051828.

La Demanderesse a découvert de manière surprenante que le choix d'un réducteur spécifique et d'un solvant particulier, mis en oeuvre en présence d'un acide organique permettait de conduire à un composé de formule (I) telle que définie précédemment sous la forme d'un seul diastéréoisomère (β, S), qui peut notamment être obtenu sans recourir à une séparation des diastéréoisomères.

L'invention concerne donc le procédé de préparation d'un composé de formule (1), tel que défini précédemment, consistant à faire réagir un composé de formule (II) : 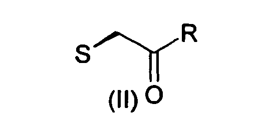 dans laquelle S et R ont la même signification que précédemment,
en solution dans un alcool secondaire linéaire, ramifié, ou cyclique et comportant de 3 à 8 atomes de carbone,
avec un réducteur comprenant : (a) un borohydrure de métal de formule MBH₄ dans laquelle M représente un métal alcalin, en présence d'un acide organique carboxylique ou sulfonique comportant de 1 à 10 atomes de carbone, ou (b) une espèce réactive ou complexe formé par l'association d'un borohydrure de métal avec un acide organique et éventuellement un alcool secondaire.

Dans un aspect avantageux de l'invention, l'alcool secondaire utilisé dans le procédé ci-dessus est l'isopropanol.

Un autre aspect avantageux de l'invention concerne un procédé de préparation tel que décrit ci-dessus dans lequel le borohydrure de métal est choisi parmi les borohydrures de sodium, de lithium ou de potassium, et plus particulièrement le borohydrure de sodium.

Les acides organiques carboxyliques convenant pour la mise en oeuvre de la présente invention peuvent être notamment choisis parmi les acides aliphatiques ayant de 1 à 10 atomes de carbone, plus particulièrement les acides acétique, propanoïque ou butanoïque, et les acides aromatiques ayant notamment de 6 à 10 atomes de carbone, en particulier l'acide benzoïque.

Les acides organiques sulfoniques convenant pour la mise en oeuvre de la présente invention sont choisis parmi les acides aliphatiques ayant de 1 à 10 atomes de carbone, plus particulièrement les acides méthanesulfonique ou trifluorométhanesulfonique, et les acides aromatiques ayant notamment de 6 à 10 atomes de carbone, en particulier l'acide para-toluènesulfonique.

L'acide organique préféré pour la mise en ouvre du procédé de préparation des composés de formule (I) tel que défini précédemment est l'acide acétique.

Alternativement et de façon particulièrement efficace, la réaction de réduction du composé de formule (II) telle que définie précédemment peut être effectuée, lorsqu'elle est disponible, à l'aide de l'espèce réactive ou complexe formé par l'association du borohydrure de métal avec l'acide organique et éventuellement alcool secondaire . On pourra citer notamment le triacétoxyborohydrure de sodium de formule NaBH(OAc)₃, qui correspond à l'association d'un équivalent molaire de borohydrure de sodium avec 3 équivalents molaires d'acide acétique.

Dans un aspect avantageux de l'invention, le rapport molaire entre l'acide organique et le borohydrure de métal mis en oeuvre dans le procédé ci-dessus est d'au moins 3.

Dans un autre aspect particulier de l'invention, le rapport entre l'alcool secondaire et l'acide organique varie entre 1/4 et 20/1 en volume, et sera avantageusement ajusté à une valeur égale à 9/2 en volume.

Des composés de formule (II) préféré pour la mise en oeuvre du procédé de préparation selon la présente invention sont la C-β-D-xylopyranoside-2-propanone de formule : 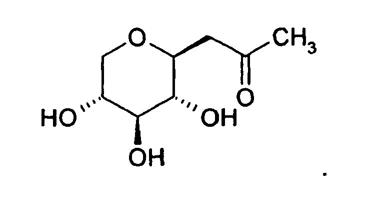 et la C-β-D-fucopyranoside-propane-2-one de formule :

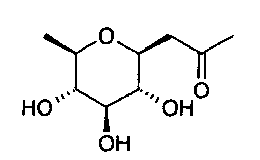

Les dérivés C-glycoside répondant à la formule (1) présentent des activités remarquables pour stimuler la synthèse des glycosaminoglycanes (GAGs) contenant un résidu D-glucosamine et/ou N-acétyl-D-glucosamine, avantageusement de l'acide hyaluronique, et/ou des protéoglycannes (PGs), avantageusement des PGs contenant de l'acide hyaluronique, par les fibroblastes et/ou les kératinocytes.

Plus précisément, il est apparu que les dérivés C-glycosides de formule (I), du fait de leur effet de stimulation de la synthèse des GAGs contenant un résidu D-glucosamine et/ou N-acétyl-D-glucosamine, avantageusement de l'acide hyaluronique, et/ou des PGs, avantageusement des PGs contenant de l'acide hyaluronique, par les fibroblastes et/ou les kératinocytes permettaient :
- de lutter contre le vieillissement de l'épiderme. Il est en effet connu que le vieillissement de l'épiderme est, pour une part importante, lié à une perte d'acide hyaluronique,
- de maintenir et/ou stimuler l'hydratation et/ou de lutter contre le dessèchement de la peau lié à une insuffisance de l'action des GAGs, en particulier de l'acide hyaluronique. Un tel dessèchement est en particulier observé sur les peaux âgées et est lié essentiellement à une perte d'acide hyaluronique,
- d'améliorer la tonicité de la peau. Il a été en effet observé que l'augmentation de la synthèse des PGs et des GAGs permettait de créer un environnement cellulaire hydraté favorable aux échanges de nutriments, d'ions, de cytokine et de facteurs de croissance sécrétés par des cellules épidermiques. Un tel environnement est favorable aussi à l'élimination des métabolites toxiques. Cet effet se traduit donc par une peau tonique et en bonne santé,
- de maintenir ou restaurer la souplesse et l'élasticité de la peau. Cet effet est lié à la stimulation de la synthèse des PGs et des GAGs qui permet de créer un environnement hydraté pour les constituants matriciels, en particulier au niveau de la jonction dermo-épidermique pour favoriser les micro-déplacements entre les éléments de cette matrice lors d'un stress mécanique. Un tel effet contribue donc à une peau plus souple et plus élastique,
- d'améliorer la minéralisation de l'épiderme, rendant ainsi la peau plus saine et améliorant sa vitalité. Cet effet est lié à l'amélioration de la synthèse des GAGs qui permet d'assurer une bonne minéralisation de l'épiderme. En effet, les GAGs, par leurs groupements chargés, peuvent fixer les ions et contribuer à l'osmolarité de l'épiderme. Là encore, une bonne minéralisation de la peau est synonyme de peau saine présentant une bonne vitalité,
- de faciliter les échanges intercellulaires. Cet effet est à rapprocher également de la stimulation de la synthèse des GAGs qui permet d'assurer une différenciation correcte de l'épiderme puisqu'une destruction de l'acide hyaluronique provoque une ouverture des espaces intercellulaires et une acantose épidermique.
   Cet effet permet d'obtenir une peau plus tonique, plus dense et plus compacte,
- d'améliorer la structure tridimensionnelle de la jonction dermo-épidermique. Ceci est également à relier à l'amélioration de la synthèse des PGs et des GAGs qui permet d'assurer l'organisation spatiale des constituants matriciels en assurant, par exemple, au niveau de la jonction dermo-épidermique, le renforcement de la liaison entre la laminine-6 et la nidogène (la nidogène est une glycoprotéine qui avec la laminine fixe les cellules endothéliales au collagène de type IV).
- de lutter contre les gerçures et l'aspect craquelé de la peau,
- de faciliter la migration des kératinocytes, permettant la formation d'une couche cornée de bonne qualité,
- de moduler l'action des facteurs de croissance et des cytokines produits par les cellules de la peau. Un tel effet permet aux cellules de disposer des signaux dont elles ont besoin pour assurer leur fonction.

Les exemples qui suivent illustrent l'invention sans en limiter la portée. Les composés sont, selon le cas, cités en noms chimiques ou en noms CTFA (International Cosmetic Ingredient Dictionary and Handbook). Les proportions sont données en pourcentage pondéral, sauf indications contraires.

### Exemple 1 : Obtention du C-β-D-xylopyranoside-2-(S)-hydroxy-propane.

A une solution de 500 mg de C-β-D-xylopyranoside-2-propanone (décrit dans l'exemple 1 la demande WO-02/051828) dans 9 ml d'isopropanol, sont ajoutés 2 ml d'acide acétique, puis rapidement 120 mg (1,2 éq.) de borohydrure de sodium en granules. Le milieu est laissé sous agitation à température ambiante pendant 30 minutes. Puis 120 mg (1,2 éq.) de borohydrure de sodium en granules sont ajoutés. Le milieu réactionnel est laissé sous agitation pendant 1 heure à température ambiante. 10 ml d'acétone sont ensuite ajoutés, et après 30 minutes d'agitation à température ambiante, le milieu réactionnel est concentré sous vide. Le résidu obtenu est purifié par chromatographie sur gel de silice pour conduire de façon sélective au composé attendu C-β-D-xylopyranoside-2-(S)-hydroxy-propane avec 95 % de rendement.

### Caractéristiques physico-chimiques du composé :

Point de fusion : 120-122 °C
Pouvoir rotatoire : - 37° (à 20°C dans le méthanol, et à une concentration [C]=1g/100ml)
¹H RMN : 1,03 (t, 3H); 1,46 (m, 1H); 1,71 (m, 1H); 2,85 (m, 1H); 2,94 (m, 1H); 2,99 (m, 2H); 3,24 (m, 1 H); 3,67 (m, 1 H); 3,77 (m, 1 H). Structure confirmée par diffraction aux rayons X .

### Exemple 2 : Obtention du C-β-D-xylopyranoside-2-(S)-hydroxy-propane

Le composé attendu est obtenu selon le procédé décrit dans l'exemple 1, en remplaçant les 2 ml d'acide acétique et le borohydrure de sodium par deux fois 1,2 équivalents de triacétoxyborohydrure de sodium (NaBH(OAc)₃).

Le produit est obtenu avec un rendement quantitatif, et sélectivement sous forme (β, S). Les caractéristiques physico-chimiques sont identiques en tous points à celles obtenues lors de l'exemple 1.

### Exemple 3 : Etude de l'effet de dérivés C-glycosides selon l'invention sur la synthèse de l'acide hyaluronique

L'étude est faite par mesure de l'incorporation de glucosamine radioactive dans la matrice néosynthétisée par des cultures de fibroblastes dermiques humains normaux. L'incorporation de glucosamine radioactive indique une néosynthèse spécifique de glycosaminoglycannes *via* une incorporation de la forme sulfatée de cette glucosamine.

Des fibroblastes dermiques normaux humains sont ensemencés en milieu de culture classique puis pré-incubés pendant 24 heures. Le milieu de culture est ensuite remplacé par les composés à tester à des concentrations de 0,3 ; 1 et 3 mM ou du milieu de culture (témoin) ou du TGFβ à 10 ng/ml (contrôle positif). Les cellules sont incubées pendant 72 heures avec ajout de ³⁵S-sulfate lors des dernières 24 heures de culture. Les GAGs de la matrice sont extraits par un tampon chaotropique puis purifiés par chromatographie d'échange d'ions (adsorption des molécules anioniques sur billes de Q-Sépharose dans des conditions de forte stringence, suivie d'une désorption des molécules peu et moyennement anioniques avec de l'urée). La radioactivité incorporée dans les molécules très cationiques restées sur le support est comptée par scintillation liquide.

Les résultats sont exprimés en pourcentage par rapport au témoin :

| **Composé testé** | **Concentration** | **cpm** | **sd** | **n** | **%/témoin** | **p** |
|---|---|---|---|---|---|---|
| Témoin | - | 5010 | 543 | 6 | 100 | - |
| TGFβ | 10 ng/ml | 9625 | 1137 | 6 | 192 | p<0.01 |
| Exemple comparatif* | 3 mM | 31735 | 799 | 3 | 633 | p<0.01 |
| | 1 mM | 20620 | 1969 | 3 | 412 | p<0.01 |
| | 0.3 mM | 10678 | 748 | 3 | 213 | p<0.01 |
| **Exemple 1** | 3 mM | 40770 | 1613 | 3 | 814 | p<0.01 |
| | 1 mM | 30943 | 588 | 3 | 618 | p<0.01 |
| | 0.3 mM | 16276 | 1195 | 3 | 325 | p<0.01 |

| | | | | | | |
|---|---|---|---|---|---|---|
| (*) : l'exemple comparatif est le composé décrit dans l'exemple 4 de la demande WO-02/051828, sous la forme d'un mélange racémique (β, S) (β, R). | | | | | | |

Les valeurs mesurées sont données en coups par minute (cpm)
sd : déviation standard
p : intervalle de confiance
n : replicates

Ces résultats indiquent que les composés de formule (I) de la présente invention possédant une conformation (β, S), stimulent la néosynthèse des GAGs de façon bien supérieure à la stimulation obtenue avec les composés de l'art antérieur qui se présentent sous forme de mélange, montrant ainsi la supériorité de l'un des deux diastéréoisomères sur l'autre.

### Exemple 5 : Préparation du C-β-D-fucopyranoside-2-(S)-hydroxy-propane.

### a) Préparation du C-β-D-fucopyranoside-propane-2-one :

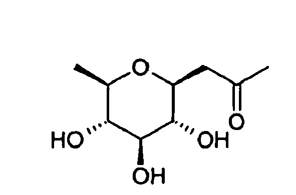

Dans un tricol de 50 ml muni d'un réfrigérant, d'un thermomètre ainsi que d'une agitation magnétique, on dissout 2g de D-fucose dans 20 mL l'eau puis on additionne la penta-2,4-dione (1.51 g, 1.2 eq) et le bicarbonate de sodium (1.53g, 1.5 eq). Le mélange est agité pendant 18 heures à 90°C. Le milieu réactionnel est ensuite lavé avec du dichlorométhane et concentré sous vide. L'huile marron obtenue est purifiée par chromatographie sur gel de silice pour conduire au composé attendu C-D-fucopyranoside-propane-2-one avec 92 % de rendement (2.3g).
Spectrométrie de masse conforme

### b) Obtention du C-β-D-fucopyranoside-2-(S)-hydroxy-propane :

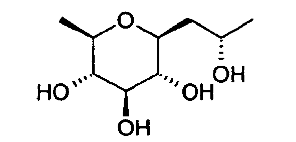

A une solution de 2.3 g de C-β-D-fucopyranoside-2-propanone dans 33 ml d'isopropanol, sont ajoutés 7.5 ml d'acide acétique, puis rapidement 510 mg (1,2 éq.) de borohydrure de sodium en granules. Le milieu est laissé sous agitation à température ambiante pendant 30 minutes. Puis 510 mg (1,2 éq.) de borohydrure de sodium en granules sont ajoutés. Le milieu réactionnel est laissé sous agitation pendant 8 heures à température ambiante. 10 ml d'acétone sont ensuite ajoutés, et après 12 heures d'agitation à température ambiante, le milieu réactionnel est concentré sous vide. Le résidu obtenu est purifié par chromatographie sur gel de silice pour conduire de façon sélective au composé attendu C-β-D-fucopyranoside-2-(S)-hydroxy-propane avec 65 % de rendement
RMN (500MHz) et Spectrométrie de masse conformes au produit attendu.

### Exemple 4 : Compositions selon l'invention

### Composition 1 : Crème Huile dans Eau (H/E)

| | |
|---|---|
| Monostéarate de glycérol | 6,0% |
| Alcool stéarylique | 4,0% |
| Huile de vaseline | 10,0% |
| Huile de silicone | 5,0% |
| C-β-D-xylopyranoside-2-(S)-hydroxy-propane | 10,0% |
| Glycérine | 8,0% |
| Polymère carboxyvinylique type Carbopol | 0,3% |
| Conservateurs | 0,4% |
| Parfum | 0,5% |
| Triéthanolamine | 0,3% |
| Eau qsp | 100% |

### Composition 2 : Crème Eau dans Huile (E/H)

| | |
|---|---|
| Octyl dodécanol | 10,0% |
| Stéarate de magnésium | 4,0% |
| Cire d'abeille naturelle | 5,0% |
| Sesquioléate de sorbitan | 4,5% |
| Mono et distéarate de glycérol et stéarate de potassium | 1,0% |
| Huile de vaseline | 22,0% |
| Huile de Jojoba | 4,0% |
| C-β-D-xylopyranoside-2-(S)-hydroxy-propane | 2,5% |
| Conservateurs | 0,4% |
| Parfum | 0,6% |
| Eau qsp | 100% |

### Composition 3 : Crème Huile dans Eau (H/E)

| | |
|---|---|
| Monostéarate de glycérol | 6,0% |
| Alcool stéarylique | 4,0% |
| Huile de vaseline | 10,0% |
| Huile de silicone | 5,0% |
| C-β-D-fucopyranoside-2-(S)-hydroxy-propane | 10,0% |
| Glycérine | 8,0% |
| Polymère carboxyvinylique type Carbopol | 0,3% |
| Conservateurs | 0,4% |
| Parfum | 0,5% |
| Triéthanolamine | 0,3% |
| Eau qsp | 100% |

## Revendications

1. Procédé de préparation d'un composé de formule (I') suivante : 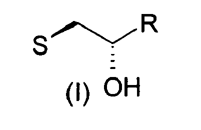 dans laquelle,
- S représente un monosaccharide, sous forme pyranose et/ou furanose et de série D, ledit monosaccharide présentant au moins une fonction hydroxyle obligatoirement libre,
- la liaison 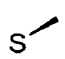 représente une liaison de configuration β,
- R représente une chaîne alkyle, linéaire ou ramifiée, comprenant de 1 à 6 atomes de carbone,
ainsi que leurs sels physiologiquement acceptable,
à l'exception des composés C-β-D-glycopyranoside-2-(S),3-dihidroxypropane et C-β-D-(2-déoxyglycopyranoside)-2-(S),3-dihydroxypropane,
consistant à faire réagir un composé de formule (II) : 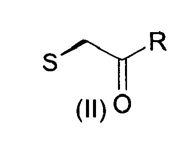 dans laquelle S et R ont la même signification que ceux de la formule (I),
en solution dans un alcool secondaire linéaire, ramifié ou cyclique, et comportant de 3 à 8 atomes de carbone,
avec un réducteur comprenant : (a) un borohydrure de métal de formule MBH₄ dans laquelle M représente un métal alcalin, en présence d'un acide organique carboxylique ou sulfonique comportant de 1 à 10 atomes de carbone, ou (b) une espèce réactive ou complexe formé par l'association d'un borohydrure de métal avec un acide organique et éventuellement un alcool secondaire.

2. Procédé selon la revendication précédente, **caractérisé en ce que** l'alcool secondaire est l'isopropanol.

3. Procédé selon l'une des revendications précédentes, **caractérisé en ce que** l'acide organique est choisi parmi l'acide acétique, l'acide propanoïque, l'acide butanoïque, l'acide benzoïque, l'acide méthanesulfonique, l'acide trifluorométhanesulfonique et l'acide para-toluènesulfonique.

4. Procédé selon l'une quelconque des revendications précédentes, **caractérisé en ce que** l'acide organique est l'acide acétique.

5. Procédé selon l'une quelconque des revendications précédentes, **caractérisé en ce que** le borohydrure de métal est choisi parmi le borohydrure de sodium, le borohydrure de potassium, et le borohydrure de lithium.

6. Procédé selon l'une quelconque des revendications précédentes, **caractérisé en ce que** le borohydrure de métal est le borohydrure de sodium.

7. Procédé selon l'une quelconque des revendications précédentes, **caractérisé en ce que** le rapport molaire entre l'acide organique et le borohydrure de métal est d'au moins 3.

8. Procédé selon l'une quelconque des revendications précédentes, **caractérisé en ce que** le rapport entre l'alcool secondaire et l'acide organique varie entre 1/4 et 20/1 en volume.

9. Procédé selon la revendication précédente, **caractérisée en ce que** le rapport entre l'alcool secondaire et l'acide organique est égal à 9/2 en volume.

10. Procédé selon la revendication 1, **caractérisé en ce que** le réducteur est formé de l'espèce réactive ou complexe formée par l'association borohydrure de métal et acide organique et éventuellement alcool secondaire.

11. Procédé selon la revendication précédente, **caractérisé en ce que** le réducteur est le triacétoxyborohydrure de sodium.

12. Procédé selon l'une quelconque des revendications précédentes, **caractérisé en ce que** le groupement R représente une chaîne alkyle linéaire ou ramifiée comprenant de 1 à 4 atomes de carbone.

13. Procédé selon l'une quelconque des revendications précédentes, **caractérisé en ce que** le groupement R représente une chaîne alkyle linéaire ou ramifiée comprenant de 1 à 3 atomes de carbone.

14. Procédé selon l'une quelconque des revendications précédentes, **caractérisé en ce que** le groupement R représente une chaîne alkyle linéaire comprenant de 1 à 2 atomes de carbone.

15. Procédé selon l'une quelconque des revendications précédentes, **caractérisé en ce que** le groupement R représente un groupement méthyle.

16. Procédé selon l'une quelconque des revendications précédentes, **caractérisé en ce que** S représente un monosaccharide dont la fonction hydroxyle en position 3 est libre.

17. Procédé selon l'une quelconque des revendications précédentes, **caractérisé en ce que** S représente un monosaccharide choisi parmi le D-glucose, le D-galactose, le D-mannose, le D-xylose, le D-lyxose, l'acide D-glucuronique, l'acide D-galacturonique, l'acide D-iduronique.

18. Procédé selon l'une quelconque des revendications précédentes, **caractérisé en ce que** S représente le D-xylose.

19. Procédé selon l'une quelconque des revendications précédentes, **caractérisé en ce que** le composé de formule (II) est la C-β-D-xylopyranoside-2-propanone.

20. Procédé selon l'une quelconque des revendications précédentes, **caractérisé en ce que** le composé (1) synthétisé est le C-β-D-xylopyranoside-2-(S)-hydroxy-propane ou le C-β-D-fucopyranoside-2-(S)-hydroxy-propane.

## Claims

1. Process for preparing a compound of formula (I') below: 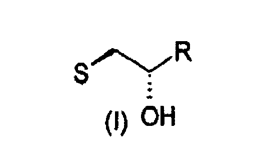 in which,
- S represents a monosaccharide, in pyranose and/or furanose form and of D series, said monosaccharide having at least one hydroxyl function that must be free,
- the bond S' represents a bond in the β-configuration,
- R represents a linear or branched alkyl chain containing from 1 to 6 carbon atoms,
and also the physiologically acceptable salts thereof,
with the exception of the compounds C-β-D-glyco-pyranoside-2-(S),3-dihydroxypropane and C-β-D-(2-deoxyglycopyranoside)-2-(S),3-dihydroxypropane, consisting in reacting a compound of formula (II): 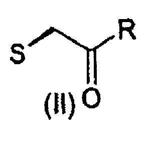 in which S and R have the same meaning as those of formula (I),
in solution in a linear, branched or cyclic secondary alcohol containing from 3 to 8 carbon atoms,
with a reducing agent comprising: (a) a metal borohydride of formula MBH₄ in which M represents an alkaline metal, in the presence of an organic carboxylic or sulphonic acid containing from 1 to 10 carbon atoms, or (b) a reactive species or complex formed by the association of a metal borohydride with an organic acid and, optionally, a secondary alcohol.

2. Process according to the preceding claim, **characterized in that** the secondary alcohol is isopropanol.

3. Process according to one of the preceding claims, **characterized in that** the organic acid is chosen from acetic acid, propanoic acid, butanoic acid, benzoic acid, methanesulphonic acid, trifluoromethanesulphonic acid and para-toluenesulphonic acid.

4. Process according to any one of the preceding claims, **characterized in that** the organic acid is acetic acid.

5. Process according to any one of the preceding claims, **characterized in that** the metal borohydride is chosen from sodium borohydride, potassium borohydride and lithium borohydride.

6. Process according to any one of the preceding claims, **characterized in that** the metal borohydride is sodium borohydride.

7. Process according to any one of the preceding claims, **characterized in that** the molar ratio of the organic acid to the metal borohydride is at least 3.

8. Process according to any one of the preceding claims, **characterized in that** the ratio of the secondary alcohol to the organic acid ranges between 1/4 and 20/1 by volume.

9. Process according to the preceding claim, **characterized in that** the ratio of the secondary alcohol to the organic acid is equal to 9/2 by volume.

10. Process according to Claim 1, **characterized in that** the reducing agent is made up of the reactive species or complex formed by the association of metal borohydride and organic acid and, optionally, secondary alcohol.

11. Process according to the preceding claim, **characterized in that** the reducing agent is sodium triacetoxyborohydride.

12. Process according to any one of the preceding claims, **characterized in that** the group R represents a linear or branched alkyl chain containing from 1 to 4 carbon atoms.

13. Process according to any one of the preceding claims, **characterized in that** the group R represents a linear or branched alkyl chain containing from 1 to 3 carbon atoms.

14. Process according to any one of the preceding claims, **characterized in that** the group R represents a linear alkyl chain containing 1 or 2 carbon atoms.

15. Process according to any one of the preceding claims, **characterized in that** the group R represents a methyl group.

16. Process according to any one of the preceding claims, **characterized in that** S represents a monosaccharide of which the hydroxyl function in the 3-position is free.

17. Process according to any one of the preceding claims, **characterized in that** S represents a monosaccharide chosen from D-glucose, D-galactose, D-mannose, D-xylose, D-lyxose, D-glucuronic acid, D-galacturonic acid and D-iduronic acid.

18. Process according to any one of the preceding claims, **characterized in that** S represents D-xylose.

19. Process according to any one of the preceding claims, **characterized in that** the compound of formula (II) is C-P-D-xylopyranoside-2-propanone.

20. Process according to any one of the preceding claims, **characterized in that** the compound (I) synthesized is C-β-D-xylopyranoside-2-(S)-hydroxypropane or C-β-D-fucopyranoside-2-(S)-hydroxypropane.

## Patentansprüche

1. Verfahren zur Herstellung einer Verbindung der folgenden Formel (I'): 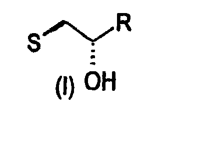 wobei in der Formel:
- S ein Monosaccharid in Pyranoseform und/oder Furanoseform der D-Reihe bedeutet, wobei das Monosaccharid mindestens eine zwingend in freier Form vorliegende Hydroxyfunktion aufweist,
- die Bindung 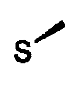 eine Bindung der Konfiguration β bedeutet,
- R eine geradkettige oder verzweigte Alkylgruppe mit 1 bis 6 Kohlenstoffatomen ist,
sowie ihre physiologisch akzeptablen Salze,
wobei die Verbindungen C-β-D-Glycopyranosid-2-(S),3-di-hydroxypropan und C-β-D-(2-deoxyglycopyranosid)-2-(S),3-di-hydroxypropan ausgenommen sind,
das darin besteht, eine Verbindung der Formel (II): 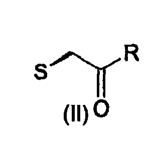 worin S und R die für Formel (I) angegebenen Bedeutungen aufweisen,
in Lösung in einem linearen, verzweigten oder cyclischen sekundären Alkohol mit 3 bis 8 Kohlenstoffatomen mit einem Reduktionsmittel umzusetzen, das umfasst: (a) ein Metallborhydrid der Formel MBH₄, wobei M ein Alkalimetall ist, in Gegenwart einer organischen Carbonsäure oder Sulfonsäure mit 1 bis 10 Kohlenstoffatomen oder (b) eine reaktive Spezies oder einen Komplex, der durch die Kombination eines Metallborhydrids mit einer organischen Säure und gegebenenfalls mit einem sekundären Alkohol gebildet wird.

2. Verfahren nach dem vorhergehenden Anspruch, **dadurch gekennzeichnet, dass** der sekundäre Alkohol das Isopropanol ist.

3. Verfahren nach einem der vorhergehenden Ansprüche, **dadurch gekennzeichnet, dass** die organische Säure unter Essigsäure, Propansäure, Butansäure, Benzoesäure, Methansulfonsäure, Trifluormethansulfonsäure und para-Toluolsulfonsäure ausgewählt ist.

4. Verfahren nach einem der vorhergehenden Ansprüche, **dadurch gekennzeichnet, dass** die organische Säure die Essigsäure ist.

5. Verfahren nach einem der vorhergehenden Ansprüche, **dadurch gekennzeichnet, dass** das Metallborhydrid unter Natriumborhydrid, Kaliumborhydrid und Lithiumborhydrid ausgewählt ist.

6. Verfahren nach einem der vorhergehenden Ansprüche, **dadurch gekennzeichnet, dass** das Metallborhydrid das Natriumborhydrid ist.

7. Verfahren nach einem der vorhergehenden Ansprüche, **dadurch gekennzeichnet, dass** das Molverhältnis der organischen Säure und des Metallborhydrids mindestens 3 beträgt.

8. Verfahren nach einem der vorhergehenden Ansprüche, **dadurch gekennzeichnet, dass** das Verhältnis des sekundären Alkohols und der organischen Säure im Bereich von 1/4 bis 20/ 1 (auf das Volumen bezogen) liegt.

9. Verfahren nach dem vorhergehenden Anspruch, **dadurch gekennzeichnet, dass** das Verhältnis von sekundärem Alkohol und organischer Säure 9/2 (auf das Volumen bezogen) beträgt.

10. Verfahren nach Anspruch 1, **dadurch gekennzeichnet, dass** das Reduktionsmittel aus einer reaktiven Spezies oder einem Komplex gebildet ist, der durch die Kombination eines Metallborhydrids und einer organischen Säure und gegebenenfalls eines sekundären Alkohols gebildet wird.

11. Verfahren nach dem vorhergehenden Anspruch, **dadurch gekennzeichnet, dass** das Reduktionsmittel das Natriumtriacetoxyborhydrid ist.

12. Verfahren nach einem der vorhergehenden Ansprüche, **dadurch gekennzeichnet, dass** die Gruppe R eine geradkettige oder verzweigte Alkylkette mit 1 bis 4 Kohlenstoffatomen bedeutet.

13. Verfahren nach einem der vorhergehenden Ansprüche, **dadurch gekennzeichnet, dass** die Gruppe R eine geradkettige oder verzweigte Alkylkette mit 1 bis 3 Kohlenstoffatomen bedeutet.

14. Verfahren nach einem vorhergehenden Ansprüche, **dadurch gekennzeichnet, dass** die Gruppe R eine geradkettige Alkylkette mit 1 bis 2 Kohlenstoffatomen bedeutet.

15. Verfahren nach einem vorhergehenden Ansprüche, **dadurch gekennzeichnet, dass** die Gruppe R die Methylgruppe bedeutet.

16. Verfahren nach einem vorhergehenden Ansprüche, **dadurch gekennzeichnet, dass** S ein Monosaccharid ist, dessen Hydroxyfunktion in 3-Stellung frei vorliegt.

17. Verfahren nach einem der vorhergehenden Ansprüche, **dadurch gekennzeichnet, dass** S ein Monosaccharid bedeutet, das unter D-Glucose, D-Galactose, D-Mannose, D-Xylose, D-Lyxose, D-Glucuronsäure, D-Galacturonsäure oder D-Iduronsäure ausgewählt ist.

18. Verfahren nach einem vorhergehenden Ansprüche, **dadurch gekennzeichnet, dass** S D-Xylose bedeutet.

19. Verfahren nach einem vorhergehenden Ansprüche, **dadurch gekennzeichnet, dass** die Verbindung der Formel (II) das C-β-D-Xylopyranosid-2-propanon ist.

20. Verfahren nach einem vorhergehenden Ansprüche, **dadurch gekennzeichnet, dass** die synthetisierte Verbindung (I) das C-β-D-Xylopyranosid-2-(S)-hydroxypropan oder das C-β-D-Fuco-pyranosid-2-(S)-hydroxypropan ist.
